# EUROPÄISCHE PATENTSCHRIFT

(11) **EP 1 702 578 B1**
(45) Veröffentlichungstag und Bekanntmachung des Hinweises auf die Patenterteilung: **29.12.2010**
(21) Anmeldenummer: 05006034.2
(22) Anmeldetag: 18.03.2005
(51) Int. Cl.: A61B 18/20

(54) **Vorrichtung für die Lichtbehandlung der Haut**
Apparatus for light treatment of skin
Dispositif de traitement lumineux de la peau

(43) Veröffentlichungstag der Anmeldung: 20.09.2006
(73) Patentinhaber: Quantel Derma GmbH, 91058 Erlangen (DE)
(72) Erfinder: Fischer, Dietmar, Dr., 91056 Erlangen (DE); Schmidt, Udo, 90562 Heroldsberg (DE)
(74) Vertreter: von Hellfeld, Axel

(56) Entgegenhaltungen:
- WO-A-02/32336
- WO-A-02/053050
- WO-A-02/089688
- WO-A-2004/000419
- WO-A-2005/011797
- US-A- 5 651 784
- US-A- 5 814 041
- US-A- 5 978 541

## Beschreibung

Die Erfindung betrifft eine Vorrichtung für die Lichtbehandlung der Haut und insbesondere eine Vorrichtung zum Entfernen von Hautpigmentierungen.

Elektromagnetische Strahlung, nachfolgend als "Licht" bezeichnet, wobei dieser Begriff auch Strahlung mit Wellenlängen erfassen soll, die außerhalb des für das menschliche Auge sichtbaren Bereiches liegen, hat eine Vielzahl von therapeutischen und/oder kosmetischen Anwendungen zur Behandlung der menschlichen Haut gefunden. Üblicherweise werden bei solchen Behandlungen großflächige Bereiche gleichzeitig bestrahlt, d.h. Bereiche im Bereich von Quadratmillimetern oder gar Quadratzentimetern.

Der Stand der Technik kennt auch Vorrichtungen und Verfahren, bei denen nur kleine Hautbereiche mit Abmessungen von 10 bis 1000 µm jeweils bestrahlt werden, vgl. WO 2004/037068 A2 und WO 2004/037069 A2. Dort werden mit einer Vielzahl von Lichtquellen jeweils "Spots" (Lichtflecken) auf die Haut aufgebracht und bei hoher Strahlungsintensität werden Löcher in die Haut gezielt "eingebrannt". Der Abstand der Löcher beträgt zwischen 30 und 2000 µm und es ist auch vorgesehen, die am Rande der bestrahlten Fläche liegenden Spots in ihrer Spot-Dichte zu verringern, um die Strahlungswirkung im Übergangsbereich zu der nicht bestrahlten Hautfläche zu reduzieren.

Allerdings kennt dieser Stand der Technik in Bezug auf die einzelnen Strahlungsflecken (Spots) selbst keine besondere Steuerung der Verteilung der Lichtintensität im Fleck und an seinen Rändern. Vielmehr hat dort die Strahlungsintensität entlang einem Diagonalschnitt durch einen Strahlungsfleck keinen besonders gesteuerten Verlauf und somit hat die Strahlung im Fleck die volle Intensität und außerhalb des Fleckes keine Intensität. Besonders an den Rändern der behandelten Punkte ("Spots") ist es bei diesem Stand der Technik sehr schwierig, sanft-kontinuierliche Übergänge der Strahlungsintensität zu erzielen. Auch hat dieser Stand der Technik den Nachteil, dass bei Erzeugen einer Vielzahl von künstlichen kleinen "Wunden" in der Haut entweder ein einzelner Spot mit einer aufwendigen Steuerung zeitlich nacheinander auf unterschiedliche Hautstellen gesetzt wird oder es müssen mit einer Vielzahl von Lichtquellen und optischen Fokussierelementen sehr aufwendige optische Einrichtungen bereitgestellt werden, um gleichzeitig eine Vielzahl von "Mikro-Wunden" in die Haut zu "brennen".

Die WO 2004/000419 A1 offenbart eine Vorrichtung zum Erzeugen eines therapeutischen photochemischen Effekts in einem Behandlungsbereich mit einer Laserlichtquelle mit einem einzigen Laserstrahl, der in ein diffraktives optisches Element gekoppelt wird.

Die WO 2005/011797 A1 offenbart ein selbstklebendes Pflaster mit einem Feld optischer Linsen. Das auf das Pflaster nahezu senkrecht auftreffende Licht wird von den Sammellinsen auf die Hornschicht der Haut separat fokussiert projiziert.

Die WO 02/089688 offenbart eine Vorrichtung zur Steuerung der Verteilung von Energie in einem Behandlungsmaterial, dessen Oberfläche durch Energie erwärmt wird, die von einer stark absorbierenden Substanz absorbiert wird und auf die Oberfläche des Behandlungsmaterials diffundiert wird. Die durch die hochabsorbierende Substanz absorbierte Energie wird in thermische Energie umgewandelt und diffundiert als thermische Energie in das Behandlungsmaterial.

Die WO 02/32336 A1 offenbart eine Bestrahlungsvorrichtung mit einem Laser dessen Lichtstrahl über zwei bewegliche Spiegel zu einer ausgewählten Region eines Patienten geführt wird. Durch eine zusätzliche Steuerung, beispielsweise der Intensität des Lasers, kann ein Ausschalten des Lasers erreicht werden, wenn dessen Strahl über eine nicht ausgewählte Region bewegt wird.

Die WO 02/053050 A1 offenbart eine Vorrichtung und ein Verfahren zur therapeutischen Behandlung der Haut eines Patienten durch Konzentrieren der Strahlung von zumindest einer ausgewählten Wellenlänge auf eine Mehrzahl ausgewählter, dreidimensional angeordneter Behandlungsbereiche. Es werden Wellenlängen im Bereich von 400 bis 1880 nm sowie 2050 bis 2350 nm vorgeschlagen. Die Behandlungsbereiche haben einen Durchmesser von 50 bis 200 µm, von 400 bis 1200 µm oder von 1000 bis 2000 µm.

Der Erfindung liegt die Aufgabe zugrunde, eine Vorrichtung für die Lichtbehandlung der Haut bereitzustellen, mit der verbesserte medizinische und/oder kosmetische Ergebnisse erreicht werden. Insbesondere soll mit der Erfindung eine narbenfreie und farbunabhängige Entfernung von Tätowierungen möglich sein und auch eine sogenannte Skinrejuvenation (Hautverjüngung).

Die Aufgabe der Erfindung wird durch eine Vorrichtung nach Anspruch 1 gelöst. Es wird eine Vorrichtungoffenbart, bei der in dem Strahlengang des von der Lichtquelle emittierten Lichtes zumindest ein intensitätsmodulierendes Element angeordnet ist, das auf und/oder in der Haut ein Lichtintensitätsprofil erzeugt mit Intensitätsmaxima und Intensitätsminima, wobei zumindest im Bereich der Intensitätsminima sich die Intensität des Lichtes in einer Vielzahl von Stufen mit jeweils relativ geringer Stufenhöhe im Vergleich zur Maximalintensität ändert.

Es erfolgt also in den Randbereichen eines Intensitätsmaximums, dort wo die Intensität sich einem Minimum, also zum Beispiel dem Wert "0" nähert, ein fließender Übergang der Strahlungsintensität, d.h. ein stufenförmiges Auslaufen jedes Spots an seinen Rändern. Entsprechendes gilt bei Strahlungslinien an deren Rändern.

Das lichtintensitätsmodulierende Element kann unterschiedliche Ausgestaltungen aufweisen, die in den abhängigen Ansprüchen beschrieben sind. So kann das Element zum Beispiel in der Art eines "Neutralfilters" (Neutral Density Filter) mit variierender Lichtabsorption ausgestalten sein. Zum Beispiel in der Art einer Glasplatte, deren Transmission aufgrund unterschiedlicher Konzentration lichtabsorbierender Stoffe ortsabhängig variiert.

Alternativ kann die in den Strahlengang gestellte teildurchlässige Platte auch in ihrer Dicke so moduliert sein, dass die Strahlung aufgrund der unterschiedlichen Wegstrecken in der Platte je nach Ort des Durchgangs durch die Platte in unterschiedlichem Maße absorbiert wird (Beersches Gesetz).

Auch mit Polarisationsfiltern, deren relative Winkelstellung in dem intensitätsmodulierenden Element unterschiedlich eingestellt ist, kann die beschriebene Intensitätsmodulation der durchgelassenen Strahlung, die letztlich auf die zu behandelnde Haut gelangt, erreicht werden. Bei dieser Ausgestaltung der Erfindung kann durch elektrische Signale die relative Winkelstellung der Polarisationsfilter in einfacher Weise verändert werden, sodass auch das erzeugt Lichtintensitätsprofil in einfacher Weise einstellbar ist.

Bevorzugt ist der Abstand zwischen den Maxima der Lichtintensitätsverteilung im Bereich von 5 bis 1000 µm.

Die Erfindung beinhaltet auch eine Vorrichtung zur Entfernung von natürlichen und künstlichen Hautpigmentierungen mit Einrichtungen zum Erzeugen von Licht mit Wellenlängen zwischen 1500 und 20000 nm in einem oder mehreren Lichtflecken mit Durchmessern zwischen 10 und 1000 µm, Einrichtungen zum Lenken solcher Lichtflecken auf die Haut, um dort eine Vielzahl von Mikro-Wunden zu erzeugen derart, dass der Abstand zwischen den die Wunden erzeugenden Lichtflecken zwischen 5 und 1000 µm beträgt.

Die vorstehend beschriebenen erfindungsgemäßen Vorrichtungen können jeweils mit einer einzigen oder auch jeweils mit unterschiedlichen Strahlungswellenlängen verwirklicht werden. Zum Beispiel mit Wellenlängen zwischen 300 nm (für zum Beispiel die Behandlung von Psoriasis und Vitiligo) und 20000 nm (CO₂-Laser). Als Lichtquelle in Betracht kommen zum Beispiel alle Arten von Lasern einschließlich Faserlaser, LEDs, sowie gepulste und andere Lampen. Die Vorrichtungen und Verfahren lassen sich mit gepulster Strahlung und mit kontinuierlicher Strahlung realisieren.

Erfindungsgemäß ist es möglich, mit einem einzigen intensitätsmodulierenden Element, zum Beispiel der oben beschriebenen Art, und mit einer einzigen (oder alternativ auch mehreren) Lichtquellen in einfacher Weise ein Intensitätsprofil der auf die Haut aufzubringenden Strahlung zu erzeugen, das gleichermaßen einem "Strahlungsintensitätsgebirge" mit Maxima und Minima entspricht.

Die von der Lichtquelle oder den Lichtquellen emittierte Strahlung kann mit unterschiedlichen Transmissionssystemen zu dem lichtintensitätsmodulierenden Element übertragen werden, zum Beispiel mit optischen Fasern oder anderen optischen Mitteln.

Mit der Erfindung ist es möglich, eine Vielzahl von Lichtspots auf oder in die Haut einzubringen, ohne dass eine Vielzahl von Lichtquellen hierfür erforderlich wäre und es kann auch auf eine aufwendige Anordnung von Fokussierelementen und zugehörigen optischen Einrichtungen (vgl. den eingangs zitierten Stand der Technik) verzichtet werden.

Das erfindungsgemäße intensitätsmodulierende Element ermöglicht quasi eine Simulation einer Vielzahl von Lichtquellen, ohne das eine solche Vielzahl erforderlich wäre. Auch ermöglicht die Erfindung eine Anpassung des Intensitätsprofils an die gewünschte medizinische oder kosmetische Anwendung in weiten Bereichen.

Nachfolgend werden Beispiele anhand der Zeichnung näher erläutert. Beispiele mit einer stetigen Intensitätsänderung sind keine Ausführungsbeipiele der Erfindung. Es zeigt:
- Figur 1: schematisch ein erstes Ausführungsbeipiel einer Vorrichtung für die Lichtbehandlung von Haut;
- Figur 2: ein lichtintensitätsmodulierendes Element und dessen Wirkung auf die Strahlung;
- Figur 3: ein anderes Ausführungsbeispiel einer Vorrichtung für die Lichtbehand- lung von Haut mit zusätzlichen Funktionen;
- Figur 4A: schematisch die Entfernung von Tätowierungen;
- Figur 4B: schematisch die Wirkung einer Bestrahlung zur Tätowierungsentfernung gemäß Figur 4A; und
- Figur 4C: das Ergebnis einer Tätowierungsentfernung gemäß den Figuren 4A und 4B.

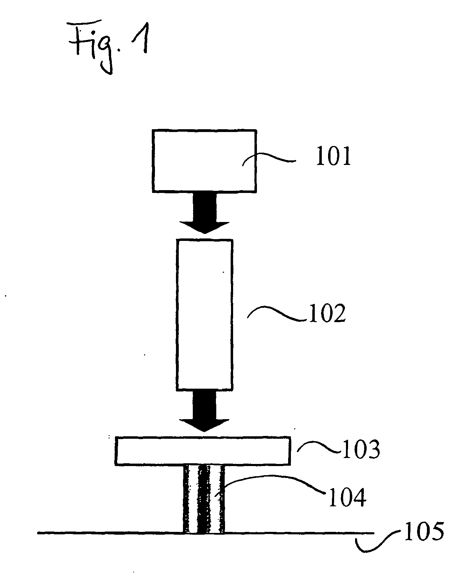

Die Lichtbehandlungsvorrichtung gemäß Figur 1 enthält eine Lichtquelle 101, für die die oben erwähnten Strahlungserzeugungsvorrichtungen in Betracht kommen. Über eine Transmissionseinrichtung 102 wird das von der Lichtquelle 101 emittierte Licht zu einem lichtintensitätsmodulierenden Element 103 übertragen, das in Figur 1 schematisch dargestellt ist. Durch dieses lichtintensitätsmodulierende Element 103 wird die auf dieses Element großflächig, zum Beispiel im Bereich von Quadratmillimetern oder Quadratzentimetern, eingestrahlte elektromagnetische Strahlung in ihrer Intensität räumlich moduliert, d.h. es entsteht ein Lichtintensitätsprofil mit Maxima und Minima in der Art eines drei-dimensionalen "Lichtintensitätsgebirges".

Diese Strahlung mit einem räumlichen, sich kontinuierlich oder diskret ändernden Lichtintensitätsprofils wird auf oder in die Haut eingebracht, zum Beispiel bis zu einer Tiefe von etwa 4 mm. Der Abstand benachbarter Minima und Maxima des Lichtintensitätsprofils liegt im Bereich zwischen 5 und 1000 µm.

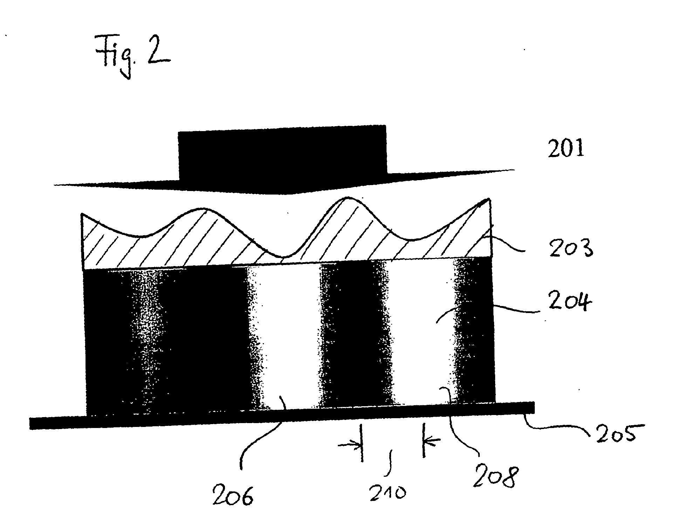

Figur 2 zeigt ein lichtintensitätsmodulierendes Element 203, das zum Beispiel für das Element 103 in Figur 1 eingesetzt werden kann.

Beim Beispiel gemäß Figur 2 trifft die von der Lichtquelle (in Figur 2 nicht gezeigt) emittierte Strahlung 201 mit großem Durchmesser ganzflächig auf das lichtintensitätsmodulierende Element 203. Das Element 203 weist in der Art eines Neutralfilters (Neutral Density Filter) Teilchen auf, die das Licht absorbieren. Diese Teilchen sind homogen in dem Element 203 verteilt. Somit wird bei Durchgang von Licht durch das Element 203 Strahlung entsprechend der Stärke des Elementes am Ort des Lichtdurchganges absorbiert. Je dicker das Element 203 an der betreffenden Stelle ist, umso mehr Licht wird dort absorbiert. Die Absorption erfolgt in erster Näherung gemäß einer Exponentialfunktion entsprechend der Stärke des Elementes 203 an der Stelle des Lichtdurchganges. Somit verlässt das lichtintensitätsmodulierende Element 203 Strahlung 204 mit einem Lichtintensitätsprofil, dass Maxima und Minima aufweist. Die Maxima 206, 208 der Lichtintensität sind in Figur 2 hell (weiß) dargestellt. Entsprechend sind Bereiche im intensitätsmodulierten Licht 204 geringer Intensität dunkler (grau bis schwarz) dargestellt. Dort, wo das lichtintensitätsmodulierende Element 203 besonders dünn ist, entstehen die Lichtintensitätsmaxima 206, 208. Dort, wo das Element 203 relativ dick ist, entstehen in Abhängigkeit von der Dicke des Elementes mehr oder weniger ausgeprägte Intensitätsprofilmimima. Überall und insbesondere in einem Randbereich, also dort, wo ein Intensitätsprofilmaximum 206, 208 in ein Intensitätsprofilminimum übergeht, ist die Intensitätsänderung nicht abrupt, sondern stetig und allmählich. Ein solcher Übergangsbereich ist in Figur 2 mit 210 gekennzeichnet. In diesem Bereich ändert sich das Lichtintensitätsprofil gemäß einer stetigen Funktion mit einem Gradienten, der insbesondere 80° oder bevorzugt 75° nicht übersteigt. Nach dem Beispiel wird ein Lichtintensitätsprofil erzeugt mit allmählichen, stetigen Übergängen, die sanfter sind als ein Lichtintensitätsprofil, das mit einer fokussierenden Linse erreicht wird, d.h. der Intensitätsgradient ist insbesondere im Übergangsbereich zum Intensitätsminimum weniger steil als bei einer fokussierenden Linse. Dabei muss die Strahlungsintensität außerhalb der den Maximalwert der Intensität aufweisenden Behandlungszone nicht notwendigerweise auf "Null" zurückgehen, vielmehr kann in diesen Übergangsbereichen die Intensität auch einen relativ geringen Wert von weniger als 10 % der Maximalintensität oder auch weniger als 5 % oder 3 % der Maximalintensität aufweisen.

Figur 2 zeigt einen Schnitt durch die betroffenen Bauelemente, also insbesondere das lichtintensitätsmodulierende Element 203. Der Ausschnitt ist schematisch, es können wesentlich mehr Maxima und Minima vorgesehen sein, als dargestellt. In anderen Schnitten als dargestellt, also zum Beispiel Schnitten senkrecht oder gewinkelt zur Papierebene, ergeben sich der Figur 2 entsprechende Bilder, d.h. die Minima und Maxima der Lichtintensitätsverteilung können in einer Draufsicht (d.h. in einer Sicht in Richtung der Strahlung 201) im wesentlichen rund sein, d.h. es entstehen Maxima und Minima in Form von "Lichtflecken" (Spots) bzw. Bereiche, in denen im wesentlichen kein Licht auf die Haut gelangt. Die Abstände zwischen den Intensitätsmaxima 206, 208 können wesentlich größer sein als die Abmessungen eines Lichtfleckes. Die Lichtflecken (Spots) selbst können zum Beispiel Durchmesser zwischen 10 und 1000 µm aufweisen.

Das lichtintensitätsmodulierende Element 203 gemäß Figur 2 definiert mit seiner Stärke die erzeugte Intensitätsverteilung auf der Haut, d.h. die jeweilige örtliche Stärke des Elementes 203 ist jeweils umgekehrt proportional zur diesem Ort entsprechenden Intensität der Strahlung auf der Haut.

Die vorstehend anhand der Figur 2 beschriebene Intensitätsverteilung kann gemäß einem Ausführungsbeispiel dahingehend abgewandelt werden, dass statt eines mathematisch gesehen stetigen Überganges eine stufenförmige Änderung der Intensität im Rand- und Übergangsbereich zum Minimalwert der Intensität vorgesehen ist. Bevorzugt sind eine Vielzahl von Stufen im Übergangsbereich vorgesehen. Beiden Beispielen (stetig und stufenförmig) ist gemeinsam, dass die Änderung der Intensität in den Randbereichen, in denen die Bestrahlung auf einen Minimalwert übergeht, nicht abrupt von 100 % auf den Minimalwert (zum Beispiel 0 %) übergeht, sondern in der beschriebenen Weise allmählich bzw. abgestuft.

Für das lichtintensitätsmodulierende Element 203 sind unterschiedliche Ausgestaltungen in Abwandlung des vorstehend anhand der Figur 2 beschriebenen Ausführungsbeispieles möglich. So können zum Beispiel mehrere Polarisationsfilter in Strahlungsrichtung hintereinander angeordnet werden und ihre relative Winkelstellung kann so variiert werden, dass das oben beschriebene Lichtintensitätsprofil erzeugt wird. Hierfür stehen auch durch elektrische Spannungen einstellbare Polarisationsfilter zur Verfügung, die dann eine einfache Anpassung des Lichtintensitätsprofils an unterschiedliche medizinische oder kosmetische Applikationen ermöglichen.

Es ist auch möglich, das lichtintensitätsmodulierende Element 203 dadurch zu realisieren, dass kleinste Kristalle in einem Träger angeordnet werden, die durch eine unterschiedliche räumliche Ausrichtung unterschiedlich Strahlung reflektieren.

Auch können halbdurchlässige Spiegel mit unterschiedlichen Transmissionen angeordnet werden. Ebenfalls in Betracht kommen verschiedene optisch anregbare Medien, zum Beispiel Farbstoffe, fluoreszierende Stoffe, Gase, Kristalle, optische Fasern, die zum Beispiel durch eine inhomogene Konzentration oder Zusammensetzungen das Lichtintensitätsprofil in der oben beschriebenen Weise mit Abständen zwischen Maxima und Minima in der Größenordnung von 5 bis 1000 µm erzeugen.

Schließlich ist es auch möglich, ein ausschließlich diskretes Lichtintensitätsprofil mit fließenden Übergängen in die nicht bestrahlten Bereiche mit einem optischen Gitter zu verwirklichen.

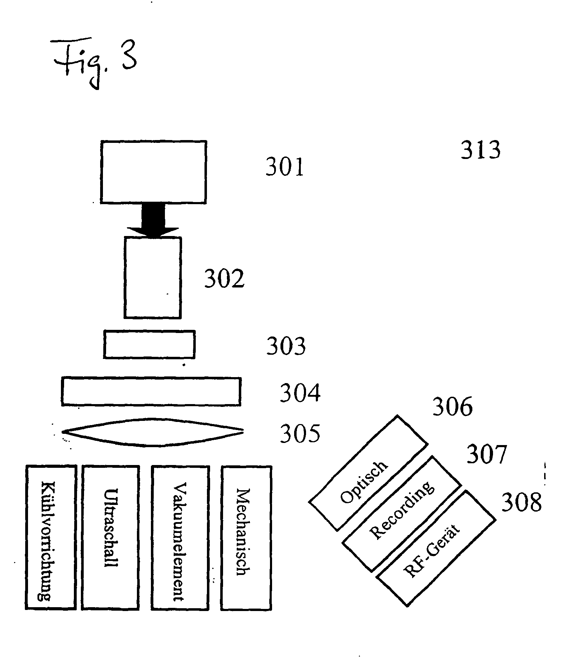

Figur 3 zeigt eine Vorrichtung für die Lichtbehandlung von Haut mit weiteren Funktionsbauteilen, die in der Figur nur schematisch dargestellt sind und dem Fachmann als solche jeweils zur Verfügung stehen, aber im Zusammenwirken mit der erfindungsgemäßen Vorrichtung besondere Vorteile ermöglichen. Die Vorrichtung gemäß Figur 3 hat wiederum eine Lichtquelle 301 und ein Transmissionssystem 302. Vor Eintritt des Lichts in das lichtintensitätsmodulierende Element 304 ist in Scanner 303 angeordnet, mit dem das Licht über eine Behandlungszone geführt werden kann.

Dem Element 304 ist ein fokussierendes Element 305 im Strahlengang nachgeschaltet.

In Bezug auf die zu behandelnde Haut können eine Kühlvorrichtung und/oder ein Ultraschallgerät und/oder eine Vakuumeinrichtung und/oder mechanische Einrichtungen zur Hautdeformation (zum Beispiel Rollen) vorgesehen sein. Solche Baugruppen sind in Figur 3 schematisch durch Blöcke dargestellt. Außerdem kann die Vorrichtung optische Elemente 306 aufweisen zur Verbesserung des Blickes auf die Behandlungszone. Diese optischen Elemente können auch Mittel zur Aufnahme und Aufzeichnung der Behandlung aufweisen, zum Beispiel Videokameras, Vergrößerungsgläser, Lupen, Kameras, Projektoren. Außerdem können auch Mittel 307 vorgesehen sein zum Aufzeichnen und zur Analyse reflektierter Strahlung. Ebenfalls einsetzbar ist ein RF-Gerät 308. Die wie oben beschrieben in die Haut eingebrachte Strahlung mit einem bestimmten Intensitätsprofil ändert das Absorptionsverhalten der Haut für RF-Strahlung, da Temperaturänderungen der Haut deren Impedanz beeinflussen. Auf diese Weise können entsprechend dem erzeugten Lichtintensitätsprofil bestimmte Bereiche der Haut gezielt erhitzt, d.h. behandelt werden.

Es ist auch möglich, die beschriebenen Vorrichtungen, insbesondere entsprechend den Figuren 1 und 2, in ein Handgerät unterzubringen, das vom Operateur bequem handhabbar ist. Dabei können Einrichtungen vorgesehen sein zur Erfassung der Bewegung des Handstückes und insbesondere die Bewegungsgeschwindigkeit des Handstückes über der Haut.

Die anhand der Figuren 1 bis 3 beschriebenen Vorrichtungen können allgemein eingesetzt werden für ablative und nicht-ablative Applikationen. Sie eignen sich zur Beseitigung von krankhaften oder unerwünschten Läsionen der Haut. Insbesondere ist es möglich, im kosmetischen Bereich Tätowierungen jeder Farbe und auch benigne pigmentierte Hautveränderungen narbenfrei zu entfernen. Letzteres soll nachfolgend erläutert werden:

Werden mit den vorstehend beschriebenen Vorrichtungen Lichtintensitäten hinreichender Stärke (Fluence) eingesetzt, dann werden in die Haut sogenannten minimalinvasive Wunden wie "Löcher" eingebrannt. Die Körperfunktionen reagieren hierauf mit einer Krustenbildung aus den tieferen Hautschichten heraus. In dieser Kruste enthalten sind neben den natürlichen gesunden Pigmenten auch die Pigmente von pigmentierten Hautveränderungen oder die künstlich eingebrachten Pigmente einer Tätowierung. Nach einer kurzen Ausheilungsphase fällt die Kruste ab und damit werden auch die darin enthaltenen Pigmente entfernt.

Mit den vorstehend beschriebenen Vorrichtungen sind bei Einstellung geeigneter Intensitäten, auch andere Applikationen möglich, wie die genannten Skinrejuvenation, also insbesondere die Glättung von Falten und Hautunebenheiten, die Reduktion der Größen von Poren, die Straffung des Gewebes (Lifting Effekt) und die generelle Vereinheitlichung der Hautpigmentierungen. Die dabei erzielbaren Effekte werden alle auf denselben Heilungsprozess zurückgeführt.

Behandelt werden können auch Warzen, Akne und alle Arten von Narben (hypertroph, atroph, hypotroph, Aknenarben).

Weitere Anwendungsgebiete sind photosensitive Hautkrankheiten, wie z.B. Psoriasis, Vitiligo, Atopische Dermatitis, Alopezia Areata, Mycosis fungoides, Depigmentierte Narben und Striae, Lichen ruber planus sowie vaskuläre Läsionen wie z.B. Couperose, Teleangiektasien, Hämangiome, Port-wine stains, Rosazea und ferner Cellulite. Ebenfalls denkbar ist eine Anwendung für die lichtunterstützte Haarentfernung.

Vorstellbar als Anwendung wäre ferner für die Materialbearbeitung die Mikrobeschriftung von Produkten und Bauteilen zur eindeutigen Kennzeichnung und zum Schutz vor illegalen Nachahmerprodukten sowie die Erzeugung feiner mechanischer und elektronischer Bauteile und 1D, 2D, 3D Strukturen im µm-Bereich durch Entfernung oder Erhitzung von Material mittels Laser.

In der Ophthalmologie könnten feine Lochstrukturen in der Hornhaut oder der Netzhaut erzeugt werden. Analog könnte natürlich auch Gewebe in diesen Strukturen entsprechend erhitzt werden.

Besonders bevorzugt können die beschriebenen erfindungsgemäßen Vorrichtungen eingesetzt werden zur Entfernung von Pigmentierungen. Dies soll nachfolgend näher beschrieben werden.

Ziel ist es, weitgehend ohne unerwünschte Nebenwirkungen Pigmentierungen zu entfernen. Solche unerwünschten Nebenwirkungen sind insbesondere Narben, postinflammatorische Hyperpigmentierungen oder Hypopigmentierungen. Auch sollen offene Wunden mit Infektionsrisiken vermieden werden. Auch lange Abheilzeiten sollen vermieden werden.

Dies wird den oben beschriebenen Vorrichtungen wie folgt erreicht.

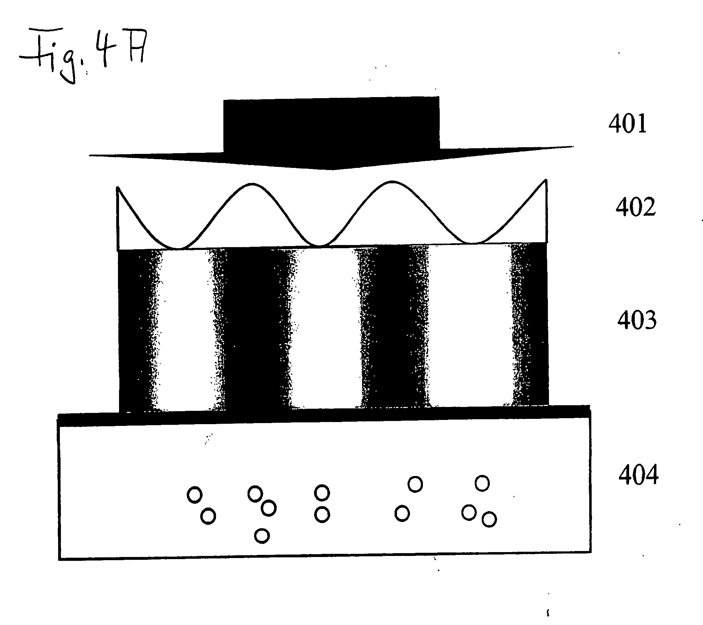

Gemäß Figur 4A wird Licht 401 durch ein lichtintensitätsmodulierendes Element 402 in der oben beschriebenen Weise so moduliert, dass Licht 403 mit einem Lichtintensitätsprofil der beschriebnen Art erzeugt wird. Dieses wird in die Haut 404 eingestrahlt. Dort sind die Pigmente in Figur 4A mit kleinen Kreisen angedeutet. Zum Beispiel wird Strahlung mit Wellenlängen zwischen 1500 und 20000 nm erzeugt und in kleine Spots mit Durchmessern zwischen 10 und 1000 µm aufgeteilt. Die Spots können dabei überlappend angeordnet sein oder auch voneinander getrennt sein, sodass zwischen den Spots nicht oder nur gering bestrahltes Gewebe gegeben ist.

Mit dieser Strahlung werden durch Gewebeabtragung (Ablation) kleine Kanäle oder Löcher in der Epidermis und Dermis erzeugt, die zum Beispiel Durchmesser im Bereich von 10 bis 1000 µm haben. Diese Kanäle bzw. Löcher reichen bis zu den Pigmenten, zum Beispiel der zu entfernenden Tätowierung. Das umliegende Gewebe außerhalb der Kanäle bzw. Löcher bleibt intakt.

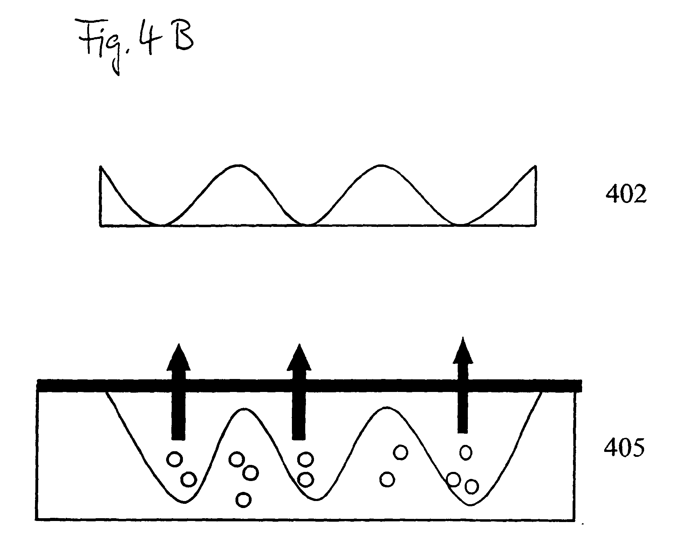

Dieser Zustand ist schematisch in Figur 4B dargestellt. Pigmente werden teils durch den Laserstrahl direkt verdampft, teils in kleinere Pigmente aufgebrochen und teils über die entstehende Kruste an die Hautoberfläche befördert. Nach einigen Tagen fällt die Kruste ab und mit ihr verschwinden auch die zu entfernenden Pigmente.

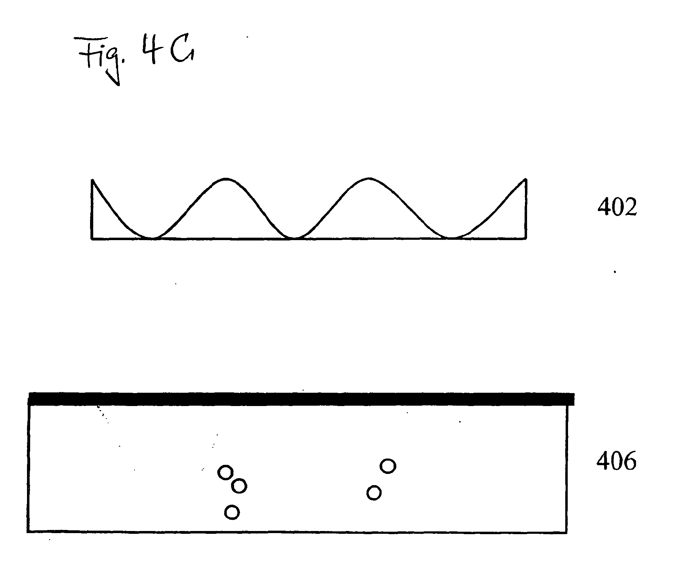

Figur 4C zeigt den danach erreichten Zustand, in dem deutlich weniger Pigmente in der Haut 406 vorliegen. In einem weiteren Behandlungszyklus analog dem vorstehend beschriebenen können auch diese restlichen Pigmente entfernt werden.

Das vorgestehend beschriebene Verfahren mit der beschriebenen Vorrichtung kann nicht nur zur Entfernung von Tätowierungen eingesetzt werden, sondern auch zur Entfernung von epidermalen und dermalen pigmentierten Läsionen, wie zum Beispiel Nävus Ota, Nävus Ito und Lentigos.

Besonders vorteilhaft bei den beschriebenen Techniken ist, dass beliebige Tätowierungsfarben und auch Farbkombinationen entfernt werden können, da die Strahlung nicht mehr von den Pigmenten absorbiert werden muss.

Das Einbringen der beschriebenen Mikro-Löcher verursacht keine sichtbaren Narben, sondern allenfalls unsichtbare Mikronarben. Auch sind die Abheilzeiten relativ kurz.

Das Risiko von Hypo- und Hyperpigmentierungen ist gering. Durch die minimale Fläche der "Wunden" bleibt auch die natürliche Barrierefunktion der Haut gegenüber Infektionen weitestgehend erhalten, sodass das Infektionsrisiko relativ gering ist.

## Patentansprüche

1. Vorrichtung für die Lichtbehandlung von Haut mit zumindest einer Lichtquelle (101), wobei in dem Strahlengang des von der Lichtquelle (101) emittierten Lichtes zumindest ein intensitätsmodulierendes Element (103) angeordnet ist, das auf und/oder in der Haut (105) ein Lichtintensitätsprofil erzeugt mit Intensitätsmaxima und Intensitätsminima, **dadurch gekennzeichnet, dass** sich zumindest im Bereich der Intensitätsminima die Intensität des Lichtes in einer Vielzahl von Stafen mit jeweils relativ geringer Stufennähe im Vergleich zur Maximal-intensität ändert.

2. Vorrichtung nach Anspruch 1, **dadurch gekennzeichnet, dass** der Abstand benachbarter Maxima (206, 208) des Lichtintensitätsprofils zwischen 5 und 1000 µm liegt.

3. Vorrichtung nach einem der Ansprüche 1 oder 2, **dadurch gekennzeichnet, dass** ein einziges lichtintensitätsmodulierendes Element (103) im Strahlengang des von der Lichtquelle (101) emittierten Lichtes angeordnet ist.

4. Vorrichtung nach einem der vorhergehenden Ansprüche, **dadurch gekennzeichnet, dass** das lichtintensitätsmodulierende Element zumindest teilweise aus lichtabsorbierendem Material besteht.

5. Vorrichtung nach einem der vorhergehenden Ansprüche, **dadurch gekennzeichnet, dass** das lichtintensitätsmodulierende Element zwei oder mehr Polarisationsfilter aufweist, deren relative Winkelstellung in solchen Bereichen variiert, dass in einem Zielgebiet das Lichtintensitätsprofil Maxima und Minima aufweist mit Abständen zwischen den Maxima im Bereich von 5 bis 1000 µm.

6. Vorrichtung nach einem der vorhergehenden Ansprüche, **dadurch gekennzeichnet, dass** das lichtintensitätsmodulierende Element zwei oder mehr Polarisationsfilter aufweist, deren relative Winkelstellung durch elektrische Signale veränderbar ist.

7. Vorrichtung nach einem der Ansprüche 1 bis 3, **dadurch gekennzeichnet, dass** das lichtintensitätsmodulierende Element Kristalle unterschiedlicher räumlicher Ausrichtung und/oder Abmessung aufweist.

8. Vorrichtung nach einem der Ansprüche 1 bis 3, **dadurch gekennzeichnet, dass** das lichtintensitätsmodulierende Element halbdurchlässige Spiegel unterschiedlicher Transmission aufweist.

9. Vorrichtung nach einem der Ansprüche 1 bis 3, **dadurch gekennzeichnet, dass** das lichtintensitätsmodulierende Element unterschiedliche optisch anregbare Medien in unterschiedlicher Konzentration und/oder Zusammensetzung aufweist.

10. Vorrichtung nach einem der Ansprüche 1 bis 3, **dadurch gekennzeichnet, dass** das lichtintensitätsmodulierende Element ein optisches Gitter ist.

11. Vorrichtung zum Entfernen von Hautpigmentierungen mit einer Vorrichtung gemäß einem der Ansprüche von 1 bis 10, mit Einrichtungen zum Erzeugen von Licht mit Wellenlängen zwischen 1500 und 20000 nm in einem oder mehreren Lichtfleck(en) mit Durchmessern zwischen 10 und 1000 µm und Einrichtungen zum Lenken solcher Lichtflecken auf die Haut, um dort eine Vielzahl von Mikro-Wunden zu erzeugen derart, dass der Abstand zwischen den die Wunden erzeugenden Lichtflecken zwischen 5 und 1000 µm beträgt.

## Claims

1. Apparatus for light treatment of the skin having at least one light source (101), wherein in the optical path of light emitted from the light source (101) at least one intensity modulating element (103) is arranged that generates on and/or in the skin (105) a light intensity profile comprising intensity maxima and intensity minima, **characterized in that** at least in the region of the intensity minima the intensity of the light changes in a multitude of steps each having a relatively low step height as compared to the maximum intensity.

2. Apparatus according to claim 1, **characterized in that** the distance of adjacent maxima (206, 208) of the light intensity profile ranges between 5 and 1000 µm.

3. Apparatus according to claim 1 or 2, **characterized in that** a single light intensity modulating element (103) is arranged in the optical path of the light emitted from the light source (101).

4. Apparatus according to one of the preceding claims, **characterized in that** the light intensity modulating element consists at least partially of light absorbing material.

5. Apparatus according to one of the preceding claims, **characterized in that** the light intensity modulating element comprises two or more polarizing filter, wherein the angular position thereof varies in such region that in a target zone the light intensity profile comprises maxima and minima having distances between the maxima in the region from 5 to 1000 µm.

6. Apparatus according to one of the preceding claims, **characterized in that** the light intensity modulating element comprises two or more polarization filter, whose relative angular position can be changed by electrical signals.

7. Apparatus according to one of claims 1 to 3, **characterized in that** the light intensity modulating element comprises crystals having different spatial orientation and/or dimensions.

8. Apparatus according to one of claims 1 to 3, **characterized in that** the light intensity modulating element comprises semi-transparent mirrors having different transmissions.

9. Apparatus according to one of claims 1 to 3, **characterized in that** the light intensity modulating element comprises different optical excitable media in different concentration and/or composition.

10. Apparatus according to one of claims 1 to 3, **characterized in that** the light intensity modulating element is an optical lattice.

11. Apparatus for removing skin pigmentations having an apparatus according to one of clams 1 to 10 comprising means for generating of light having wave lengths between 1500 and 20000 nm in one or more light spots(s) having diameters between 10 and 1000 µm and means for directing such light spots onto the skin in order to cause micro traumata such that the distance between the light spots causing the traumata ranges between 5 and 1000 µm.

## Revendications

1. Dispositif pour le traitement de la peau par la lumière ou photothérapie de la peau, comprenant au moins une source de lumière (101), dispositif dans lequel au moins un élément de modulation d'intensité (103) est agencé dans le parcours de propagation du rayonnement de la lumière émise par la source de lumière (101), cet élément de modulation d'intensité engendrant sur et/ou dans la peau (105) un profil d'intensité lumineuse présentant des maximas d'intensité et des minimas d'intensité, **caractérisé en ce qu'**au moins dans la région des minimas d'intensité, l'intensité lumineuse varie selon un grand nombre de paliers présentant chacun respectivement une hauteur de palier relativement faible comparée à l'intensité maximale.

2. Dispositif selon la revendication 1, **caractérisé en ce que** la distance d'espacement de maximas voisins (206, 208) du profil d'intensité lumineuse se situe entre 5 et 1 000 µm.

3. Dispositif selon l'une des revendications 1 ou 2, **caractérisé en ce qu'**un seul élément de modulation d'intensité lumineuse (103) est agencé dans le parcours de propagation du rayonnement de la lumière émise par la source de lumière (101).

4. Dispositif selon l'une des revendications précédentes, **caractérisé en ce que** l'élément de modulation d'intensité lumineuse est constitué au moins en partie d'un matériau absorbant la lumière.

5. Dispositif selon l'une des revendications précédentes, **caractérisé en ce que** l'élément de modulation d'intensité lumineuse comprend deux filtres polarisants ou davantage, dont la position angulaire relative varie dans des plages telles, que dans une région cible, le profil d'intensité lumineuse présente des maximas et minimas avec des distances d'espacement entre les maximas se situant entre 5 et 1 000 µm.

6. Dispositif selon l'une des revendications précédentes, **caractérisé en ce que** l'élément de modulation d'intensité lumineuse comprend deux filtres polarisants ou davantage, dont la position angulaire relative peut être modifiée par des signaux électriques.

7. Dispositif selon l'une des revendications 1 à 3, **caractérisé en ce que** l'élément de modulation d'intensité lumineuse comprend des cristaux d'orientation spatiale différente et/ou de dimension différente.

8. Dispositif selon l'une des revendications 1 à 3, **caractérisé en ce que** l'élément de modulation d'intensité lumineuse comprend des miroirs semi-transparents à transmission différente.

9. Dispositif selon l'une des revendications 1 à 3, **caractérisé en ce que** l'élément de modulation d'intensité lumineuse comprend des milieux ou agents différents pouvant être excités optiquement, dans des concentrations et/ou des compositions différentes.

10. Dispositif selon l'une des revendications 1 à 3, **caractérisé en ce que** l'élément de modulation d'intensité lumineuse est un réseau de diffraction optique.

11. Dispositif pour l'élimination de pigmentations de la peau, comprenant un dispositif selon l'une des revendications 1 à 10, et comprenant également des appareillages pour produire de la lumière avec des longueurs d'ondes entre 1 500 et 20 000 nm, sous la forme d'un ou de plusieurs spot(s) lumineux, qui présente(nt) des diamètres entre 10 et 1000 µm, l'ensemble comprenant en outre des appareillages pour diriger de tels spots lumineux sur la peau en vue d'y engendrer un grand nombre de microlésions, de façon telle que la distance entre les spots lumineux engendrant les lésions se situe entre 5 et 1 000 µm.
